# EUROPEAN PATENT SPECIFICATION

(11) **EP 4 561 428 B1**
(45) Date of publication and mention of the grant of the patent: **06.05.2026**
(21) Application number: 23749007.3
(22) Date of filing: 27.07.2023
(51) Int. Cl.: A61B 5/00, A61B 5/113, A61B 7/00, A61B 5/1455, A61B 5/11

(54) **SYSTEM FOR MONITORING SLEEPING DISORDERS**
SYSTEM ZUM ÜBERWACHEN VON SCHLAFSTÖRUNGEN
SYSTÈME POUR SURVEILLANCE DE TROUBLES DU SOMMEIL

(30) Priority: 27.07.2022 EP 22187174
(43) Date of publication of application: 04.06.2025
(73) Proprietor: Fundació Institut de Bioenginyeria de Catalunya (IBEC), 08028 Barcelona (ES); Universitat politècnica de Catalunya (UPC), 08034 Barcelona (ES)
(72) Inventor: JANÉ CAMPOS, Raimon, 08027 Barcelona (ES); FERRER LLUÍS, Ignasi, 17003 Girona (ES); CASTILLO ESCARIO, Yolanda, 08032 Barcelona (ES)
(74) Representative: Hoffmann Eitle
(86) International application number: PCT/EP2023/070832
(87) International publication number: WO 2024/023212

(56) References cited:
- WO-A1-2022/130205
- US-A1- 2014 350 355
- US-A1- 2015 374 265
- US-A1- 2019 142 625
- US-A1- 2022 133 222
- US-A1- 2022 218 293

## Description

### Technical Field

The present disclosure relates to a method for monitoring a sleeping disorder and a sleeping quality in a subject person. More particular, the present disclosure relates to corresponding method and systems that employ mobile devices, such as smartphones or other wearable devices. Some aspects may also relate to corresponding positional treatment.

### Background Art

Obstructive sleep apnea (OSA) is a prevalent sleep disorder that is related to sleepiness and accidents and is also an independent risk factor of cardiac, brain and cancer diseases. However, more than 80% of patients are late diagnosed, as conventional techniques to diagnose OSA do not provide a method for monitoring sleeping of a subject person in a reliable and also in a cost-effective way. Known methods are based on questionnaires, such as the STOP-Band and Berlin questionnaires, which are supposed to quantify the sleep quality of the patient. However, they oftentimes fail to properly determine different sleeping positions and find their relations to the sleep apnea.

Other techniques, such as polysomnography (PSG), which some regard as the golden-standard test for diagnosing sleep-related diseases, use multiple sensors, such as a nasal pressure transducer, thermistor, and thoracic and abdominal bands, for recording breathing activity, and accelerometry-based sensors, as well as cameras, for monitoring sleep position, which leads to a more objective and reliable measure. However, the PSG is cumbersome and costly; it requires the subject to sleep with multiple wires, and promotes the subject to sleep in a certain supine position, which may even worsen any disorder already present.

In addition to the above, it is well known to employ the today ubiquitous presence of mobile electronic devices, especially smartphones. The capabilities of modern smartphones have long been appreciated in many technology fields. The fact that such mobile devices do not only provide substantial processing power and high-quality user interfaces, but also sensors and detectors for sound, light, position, accelerations and forces and the like, has already led to a broad variety of applications in technical fields that do not directly relate to telephony and communication.

However, the mere existence of sensors and data acquisition and processing capabilities does not automatically yield practicable solutions. Especially in the context of health-related issues, in the field of telemedicine, telehealth, telemonitoring and mHealth, there is the need for a factual provision of well-defined, predictable and - above all - reliable solutions and related advantages.

There is therefore a need for improved methods and systems that provide reliable functionality in the context of sleeping disorders.

### Summary

The above problems are solved by the subject-matter of the independent claims. Further preferred embodiments are given by the subject-matter of the dependent claims. Moreover, further examples are provided for facilitating the understanding of the disclosure.

According to an embodiment, there is provided a method for monitoring a sleeping disorder in a subject person, the using a mobile device and comprising: collocating the mobile device with the subject person; acquiring audio signals indicative of breathing of the subject person by means of a microphone of the mobile device; acquiring accelerometer signals, by means of an accelerometer of the mobile device, to derive position, angular and movement information of the subject person from the accelerometer signals or the signals from the accelerometer; measuring thoracic movement related to respiration and sleep-disordered breathing by means of the accelerometer; identifying silence events based on the audio signals; classifying the silence events into apneas or hypopneas; identifying an apnea type of the classified apnea based on the thoracic movement; and associating to a classified silence event the respective angular information by combining the audio signals and the accelerometer signals.

According to another embodiment, there is provided a method for monitoring a sleeping quality in a subject person, the method using a mobile device and comprising: collocating the mobile device with the subject person; acquiring audio signals indicative of breathing of the subject person by means of a microphone of the mobile device; acquiring accelerometer signals, by means of an accelerometer of the mobile device, to derive position, angular and movement information of the subject person from the accelerometer signals; measuring thoracic movement related to respiration and sleep-disordered breathing by means of the accelerometer; identifying a sleep event based on the audio signals; and identifying body position at which the sleep event is produced based on the angular information.

According to another embodiment, there is provided a system for monitoring sleeping disorder and quality in a subject person, the system comprising: a mobile device comprising; a microphone configured to acquire audio signals indicative of breathing of the subject person; and an accelerometer configured to acquire accelerometer signals including position, angular and movement information of the subject person, a fixation system for collocation the mobile device with the subject person; and a pulse oximeter configured to measure oxygen saturation levels of the subject person, wherein the fixation system is composed of an elastic band and a bag to place the mobile device.

### Brief Description of the Drawings

Embodiments of the present disclosure will now be described with reference to the figures in which:
- Figure 1: shows a schematic view of a configuration for applying and embodiment;

- Figure 2: shows schematically the concepts of the position, angular and movement information related to a subject person in embodiments;

- Figure 3: shows a schematic view of a configuration of the fixation system which is for collocating the mobile device with the subject person;
- Figure 4: shows a schematic view of a configuration of the system for monitoring sleeping disorder and quality in a subject person;

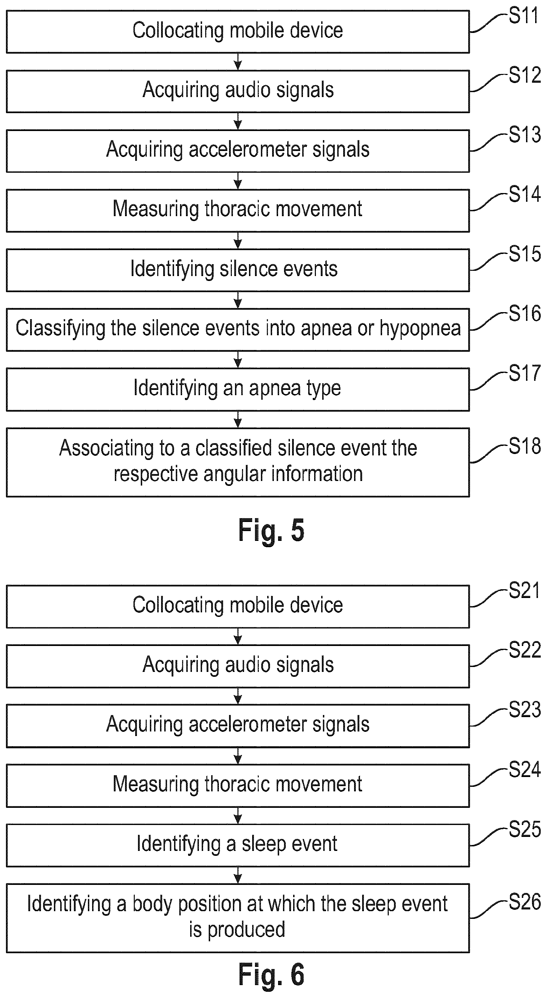

- Figure 5: shows a schematic flow chart of a general method embodiment;

- Figure 6: shows a schematic flow chart of a method for monitoring sleeping quality;

- Figure 7: shows signals recorded by the system according to an embodiment;

- Figure 8: shows apnea and hypopnea types detected by the system according to an embodiment;

- Figure 9: shows differences between nasal and oral breathing, as distinguished by an embodiment;

- Figure 10: shows cardiac activity measured by an accelerometer of the system according to an embodiment

- Figure 11: and by a classical polysomnography; and shows an example of report which can be made by the system according to an embodiment.

### Detailed Description

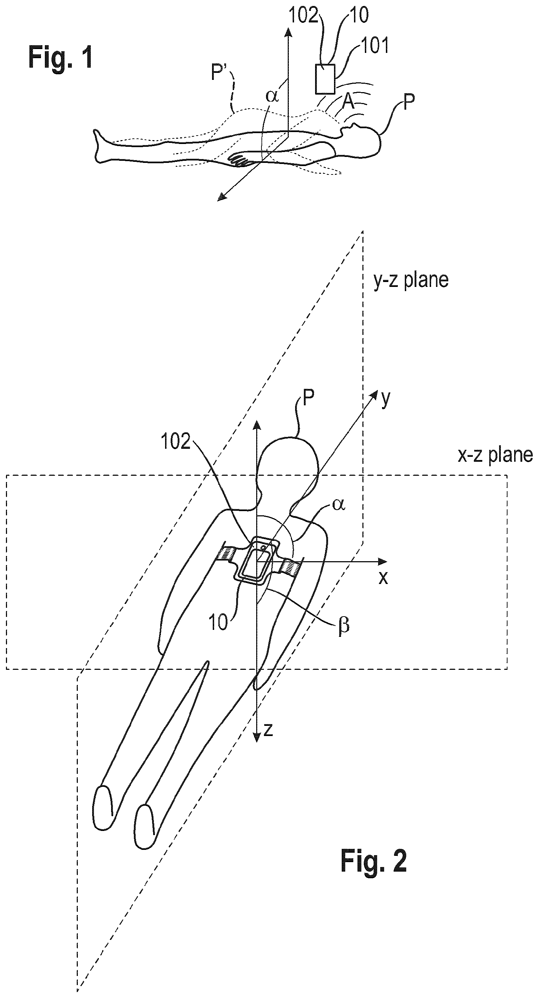

Figure 1 shows a schematic view of a configuration for applying and embodiment.

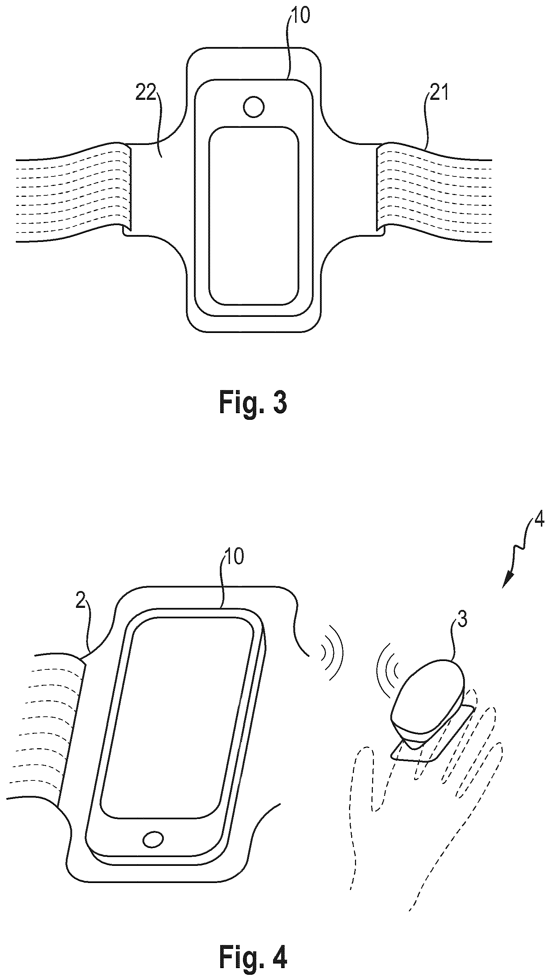

Specifically, this embodiment provides monitoring a sleeping disorder in a subject person P and the configuration considers a mobile device 10 and collocating the mobile device 10 with the subject person P. Said collocating may comprise ensuring that the mobile device 10 follows the movements by the subject person P. For example, the collocating can be obtained by securing the mobile device 10 in a pocket of apparel worn by the subject person P. Further, the collocating may be obtained by means of a fixation system 2 affixed by a strip or belt to the subject person P. The fixation system 2 is further detailed in Figure 3. Generally, said mobile devices can be implemented by any one of a mobile computing and/or communication device, a mobile phone, a smartphone, a wearable device, an electronic wristwatch, a smartwatch, and the like.

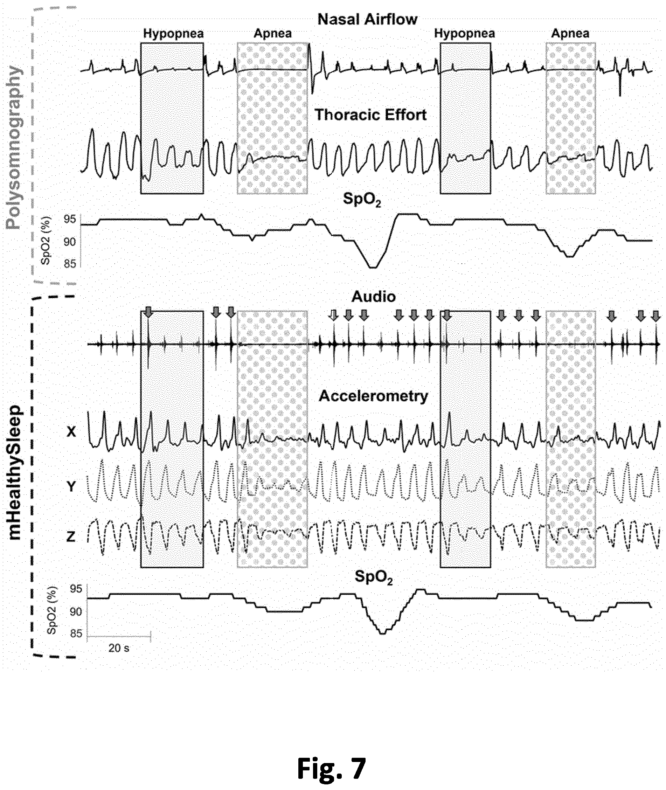

The embodiment then comprises acquiring audio signals. The audio signals indicative of breathing of the subject person P are acquired by means of a microphone 101 of the mobile device 10. For example, the audio signals A may be obtained by a built-in microphone 101 of the mobile device 10 during the overnight, with a sampling rate of 48 kHz. Examples of the audio signals A obtained by the built-in microphone 101 of the mobile device 10 during the night can be seen in Figures 7-9.

The embodiment further comprises acquiring accelerometer signals, by means of an accelerometer 102 of the mobile device 10, to derive position, angular (e.g. as depicted with α in Fig. 1) and movement information of the subject person P from the accelerometer signals. As the mobile device 10 is collocated with the subject person P, the mobile device's accelerometer 102 can advantageously detect the subject person's movement while monitoring an acute or potential sleeping disorder. Specifically, the accelerometer signals can be employed for detecting or distinguishing a supine position (as shown in Figure 1 for the subject person P or a lateral position indicated by P' in Figure 1. Although not shown in Figure 1, the accelerometer signals can also be employed for detecting or distinguishing other positions such as a prone or an upright position of the subject person P. Furthermore, a sleeping position of the subject person P is not limited to one of the pure supine, lateral, prone or upright position where the angular information assumes roughly multiples of 90°, and the sleeping position of the subject person P is not limited to the supine position. The angular information may be an angle, a plurality of angles, or triaxial angles. The angular information may also be triaxial accelerometer values, which can be derived from the accelerometer signals acquired by the accelerometer 102. The position, angular and movement information related to a subject person are further detailed in conjunction with Figure 2.

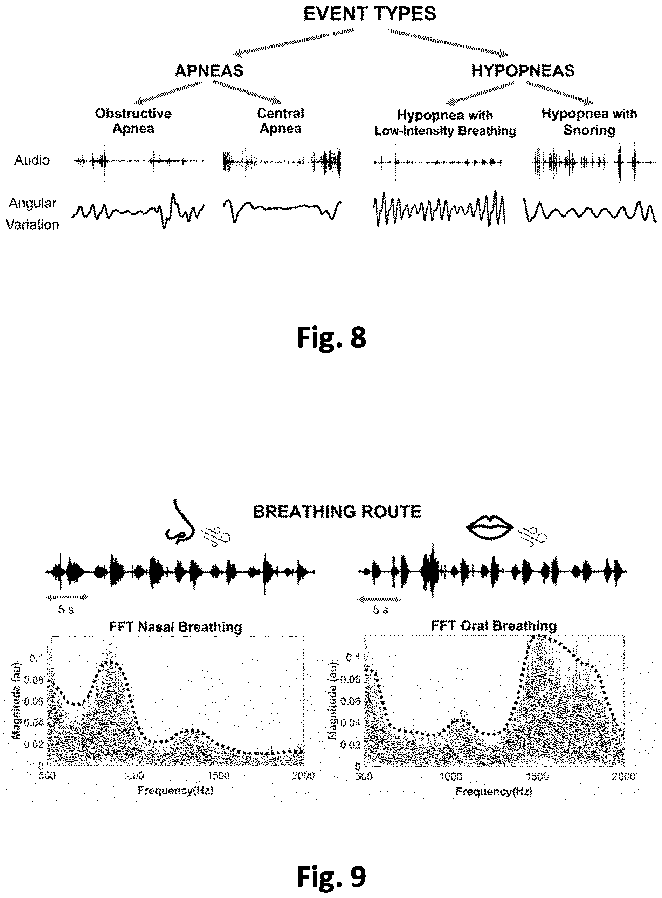

The present embodiment further comprises measuring thoracic movement related to respiration and sleep-disordered breathing by means of the accelerometer 102. This means, for example, the accelerometer 102 of the mobile device 10 collocated above the sternum of the subject person P may be used to measure repetitive vertical movements of the chest caused by breathing of the subject person P. When breathing is present in a subject person, it is possible recognize the existence of the breathing by movements of the chest as the anterior-posterior, vertical and transverse dimensions of the thorax are increased and deceased in inhalation and exhalation each, see also accelerometery of Figure 7 and angular variation of Figure 8. The collocated mobile device 10 can be used in measuring this thoracic movement related to respiration. Further, when the respiration is paused or weakened due to the sleeping disorder, the sleep-disordered breathing can be also detected as the movement of the chest may be temporarily stopped or weakened, see also angular variation shown in Figure 8.

The present embodiment further comprises identifying silence events based on the audio signals. The silence events can correspond to events which may represent sleeping disorder. For example, when the subject person P has OSA, there may be repetitive episodes of total or partial airflow reduction during sleep (i.e., apneas and hypopneas, as in Figures 7 and 8), produced by upper airway obstruction. These breathing disturbances may be recognized as silence events in the audio signals A. For the recognition of the silence events, sample entropy may be used in the calculation, as snoring and breathing present more complex patterns than silence regions, hence producing higher sample entropy values. However, the calculation used in the recognition of the silence events is not limited to a certain function.

Once the silence events are identified, the silence events are classified in apnea or hypopnea, as shown in Figure 8. In analysis of the audio signals A, if there is residual breathing activity present in the silence event, then the silence event would be classified as an event representing hypopnea, while if there was no breathing in the silence event at all, the silence event would be labelled as apnea. The residual breathing activity may be recognized by existence of low-intensity respiratory sounds recorded through the microphone 101.

The present embodiment further comprises identifying an apnea type of the classified apnea based on the thoracic movement. The accelerometer 102 may be used to identify the classified apnea into three types, which are, obstructive, central and mixed. The obstructive apnea is caused due to the upper way obstruction while the central apnea is produced when the brain does not send proper signals to the responsible muscles to control breathing. The mixed apnea is an apnea which begins as a central apnea and ends as an obstructive apnea or vice versa. Examples of the obstructive apnea and the central apnea detected by an embodiment are shown in Figure 8.

Further, the embodiment comprises associating to a classified silence event the respective angular information by combining the audio signals and the accelerometer signals. At each classified silence event, the respective angular information may be associated and be analysed to determine the positional occurrence of the sleeping disorder. Identification of the position represented by the angular information at the silent event which may represent apnea or hypopnea may be greatly helpful in some cases. For example, supine sleeping position is known to promote the occurrence of apnea compared to lateral and prone sleeping positions. This phenomenon is known as positional obstructive sleep apnea (pOSA) and is one of the most common diseases affecting sleeping quality. Therefore, revealed positional patterns of the sleeping disorder may be effectively used in monitoring a subject person with pOSA.

Figure 2 shows schematically the concepts of the position, angular information and movement information related to a subject person in embodiments.

Specifically, Figure 2 depicts a sleeping angle α and stand angle β according to x, y and z axes. As shown in Figure 2, the accelerometer 102 provides positive values when the acceleration goes from right to left (x-axis), from toe to head (y-axis) and from front to back (z-axis), thus defines the X-Z plane, where the sleep angle is calculated, and the Y-Z plane, where the stand angle is calculated. Sleeping angle α = 0° represents a pure left sleeping position, α = 90° represents a pure supine sleeping position, α = ± 180° represents pure right sleeping position and α = -90° represent pure prone sleeping position. Further, when the stand angle β = 0°, it means the position of the subject person P is headstand, a pure laying position when β = ± 90° and a standing position when β = ± 180°. Moreover, the thoracic movement caused by inhalation and exhalation of the subject person P may be explained in the Figure 2 as well. By measuring vertical movement, which may be represented by repetitive ups and downs in z-direction of the accelerometer 102 of the mobile device 10, or in other directions when the subject person P is sleeping in other positions, the thoracic movement of the subject person P may be recorded and further used in the classification of apnea types or in other applications.

Figure 3 shows a schematic view of a configuration of the fixation system 2 which is for collocating the mobile device 10 with the subject person P. Specifically, the mobile device 10 may be located in a bag 22 and attached to chest of the subject person P, or specifically over the sternum of the subject person P, with an elastic band 21.

In a further embodiment, the method comprises quantifying sleep movements and sleep position variability based on the accelerometer signals. For example, angular information derived from the accelerometer signals may be quantified as angular resolution related to the sleeping position. Further, position and/or movement information included in the accelerometer signals may be quantified as sleep position variability.

Moreover, the method further comprises detecting breathing route during sleep from the audio signals. The breathing route during sleep may be classified into oral breathing or nasal breathing, see also Figure 9. Oral breathing indicates that the subject person is breathing through the mouth whereas nasal breathing indicates that the subject person is breathing through the nose. Considering that oral breathing may be more prevalent with subjects with OSA, who have nasal obstruction that lead to increased breathing through the mouth, detecting breathing route by means of the audio signals A acquired by the mobile device 10 may be advantageous in monitoring sleeping disorder.

When the audio signal is acquired, the audio signal may be downsampled to 5 kHz, band-pass filtered between 70 and 2000 Hz and the power-line noise may be removed. If the signal is too noisy, an optimal filter or spectral subtraction may be applied to reduce interferences and improve signal quality. After that, using temporal, spectral, and time-frequency representations, apnea and hypopnea events may be identified. The spectrogram may be used as time-frequency representation with a window of 0.1s, 90% overlap and NFFT = 1024, to distinguish if there is any breathing present during the events and thus classify them into apnea or hypopnea. The Fast Fourier Transform may be calculated, and a linear envelope may be extracted, using windows of 15 Hz, to check if there is a prominent peak between 950 Hz and 2 kHz, indicative of oral breathing, as shown in Figure 9. The information extracted in this calculation may be used in further classification of hypopnea into nasal or oral breathing, and even in detecting the breathing route at any time point during the night. The percentage of time that the subject is breathing through the mouth can be calculated.

In yet another embodiment, the method further comprises acquiring oxygen saturation levels of the subject person P by means of a pulse oximeter 3 via a wireless link to the mobile device 10. The pulse oximeter is a non-invasive device for measuring blood oxygen saturation (SpO2). Apnea and hypopnea may be associated with local reductions in SpO2, which are desaturations followed by reoxygenations, as those in Figure 7. For this reason, the oxygen saturation levels may be used as a measure for identifying OSA.

Moreover, the embodiment further comprises selecting regions of interest, wherein the regions of interest are regions in the audio signals A that are followed by oxygen desaturations measured by the wireless pulse oximeter 3. When the oxygen saturation level drops more than 3%, it may be recognized as a desaturation event and used in analysis of sleeping disorder such as apnea, hypopnea and/or snoring. For a higher accuracy, the silence events may be identified only if they are followed by desaturation events measured by the wireless pulse oximeter 3 that is coupled to the mobile device 10.

In one embodiment, the method further comprises removing artefacts by considering the accelerometer signals while identifying silence events. When the subject person P moves during sleep, the noise generated during the movements may be recorded with the breathing sound in the audio signals A. Therefore, the accelerometer 102 may be used to identify movements of the subject person P and remove artefacts in identifying silence events from the audio signals A.

In one embodiment, the method further comprises classifying the silence events into apneas or hypopneas depending on existence of low-intensity respiratory sounds in the audio signals. If the low-intensity respiratory sounds exist, it can be assumed that residual breathing activity is present and the respective silence event should be classified as a hypopnea. On the other hand, if there is no breathing at all, the silence event should be labelled as an apnea. Examples of apneas with no breathing at all and hypopneas with low-intensity respiratory sounds can be found in Figures 7 and 8.

In another embodiment, the method further comprises providing the classified silence events with associated the respective angular information on the mobile device 10. The classified silence event that are associated which the respective angular information may be provided on the mobile device 10. Specifically, the classified silence events that are recorded during night may be shown in a smartphone application for an easier sleeping monitoring of the subject person P.

In another embodiment, the method further comprises outputting vibration and/or sound from the mobile device 10 based on the angular information and the oxygen saturation level and/or the audio signals. For example, when a subject person P using the mobile device 10 with the method provided in the embodiment, the method may be able to provide an alert to the subject person P in a specific position, such as supine position, which may promote occurrence of apnea compared to lateral and prone sleeping positions. For this positional treatment, the mobile device 10 is configured to output vibration and/or sound to alert the subject person P. By promoting the subject person P to change the sleeping position, sleeping in the supine position may be avoid by the positional treatment.

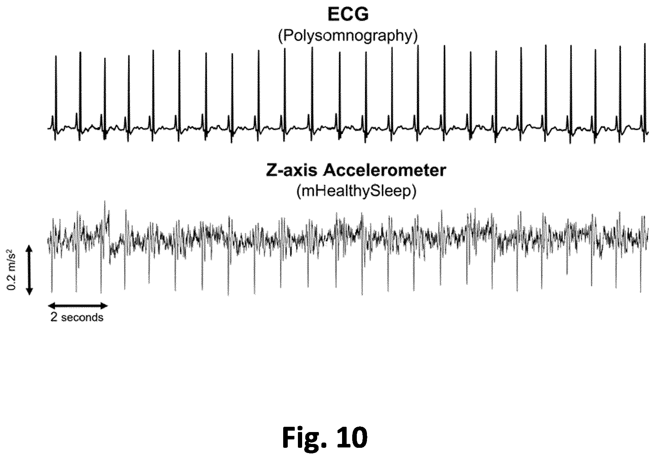

In one embodiment, the method further comprises detecting cardiac activity from the accelerometer 102, as shown in Figure 10. For example, the cardiac activity detected from the accelerometer 102 may include detection of changes of heart rate of the subject person. Then, the method further comprises associating a relationship of the changes of heart rate of the subject person and the silence events.

The disclosure may be used to monitor sleeping quality of the subject person P as well. The monitoring of the sleeping quality may be identified similarly to the monitoring of the sleeping disorders. In this embodiment, however, sleep events may be identified instead of the silence events.

For example, the method according to the embodiment comprises the same steps as in the method for monitoring a sleeping disorder until the identification of the sleep event. Then, the method according to the embodiment identifies a sleep event, including snoring episodes, based on audio signals A acquired by means of the microphone 101 of the mobile device 10. For example, arrows in Figure 7 indicate snoring episodes.

Once the sleep event is identified, a body position at which the sleep event is produced is identified based on the angular information of the subject person P, which is derived from the accelerometer signals.

The embodiment further comprises automatic detecting of multimodal features and labelling of the sleep event by multimodal features. The multimodal features are derived from the audio signals A, the accelerometer signals, and the oxygen saturation levels.

The embodiment further comprises detecting cardiac activity from the accelerometer 102 of the mobile device 10. The accelerometer signals automatically detected as a part of the multimodal features may contain higher frequency components compared to the movements from the accelerometer 102. Therefore, it is possible to detect the cardiac activity from the accelerometer 102 during the monitoring of the sleeping quality in the subject person P, as shown in Figure 10. Moreover, the embodiment may further associate a relationship of changes of heart rate of the subject person and sleep event. Furthermore, the detection of the cardiac activity may be performed in a same way as in the method for monitoring the sleeping disorder in the subject person P. In this case, the association may be made between the changes of heart rate and the silence events. However, detecting of the cardiac activity may be performed in a same way in both the monitoring of a sleeping disorder and quality.

Figure 4 shows a schematic view of a configuration of the system for monitoring sleeping disorder and quality in a subject person. In this embodiment described in the Figure 4, the system 4 comprises a mobile device 10, a fixation system 2 and a pulse oximeter 3. Although shown as a wireless pulse oximeter for in the Figure 4, the pulse oximeter 3 may be wired or wireless and is configured to measure oxygen saturation levels of the subject person P. The mobile device 10 of the system 4 comprises a microphone 101 configured to acquire audio signals A indicative of breathing of the subject person P and an accelerometer 102 configured to acquire accelerometer signals including position, angular and movement information of the subject person P. The system 4 further comprises the fixation system 2 as described in the Figure 3.

In further embodiments different multimodal features can be extracted from the audio, the accelerometer and the pulse oximeter signals. In such embodiments there is provided a method for monitoring a sleeping quality in a subject person, the method using a mobile device and comprising: collocating the mobile device with the subject person; acquiring audio signals indicative of breathing of the subject person by means of a microphone of the mobile device; acquiring accelerometer signals including position, angular and movement information of the subject person by means of an accelerometer of the mobile device; measuring thoracic movement related to respiration and sleep-disordered breathing by means of the accelerometer of the mobile device; identifying a plurality of sleep events by means of the audio signals; identifying body position at which the sleep event is produced based on the angular information; labelling of the sleep events by multimodal features; and automatic detecting of the multimodal sleep features, wherein the multimodal features are the acquired audio signals, the acquired accelerometer signals and oxygen saturation levels acquired by means of a wireless pulse oximeter via a wireless link to the mobile device.

In such embodiments the detection of multimodal features may be automatic because it can be automatically calculated by custom-made computational algorithms that may not require manual review or annotation of the signals. Several features can be extracted, including features describing the desaturation events (e.g., depth or duration of the desaturation), features related to the apneas and hypopneas (indices counting the number of apneas and/or hypopneas, duration, whether they include snoring...), snoring (from the audio signals or vibration from the accelerometer signals), features related to movement, sleep position or sleep stages, related to cardiac activity, and the like. It is noted that to "label" the sleep event should be understood in that a sleep event is annotated in the sense of identifying and classify it, e.g. in order to indicate that there is an event starting at, say, a time=1100s and ending at time=1122s and that it corresponds to an obstructive apnea. Further, "multimodal features" are to be understood as more general and "multimodal sleep features" may indicate that they are related to aspects of sleep.

Figure 7 shows signals recorded by the system according to an embodiment. Specifically, Figure 7 shows a comparison between recorded signals from a classical polysomnography and the system. Among different signals which can be recorded from the classical polysomnography, nasal airflow, thoracic effort and SpO2 channels are shown in the upper plot of Figure 7. In the system, audio, accelerometry and SpO2 have been measured comparatively, see the lower plot of Figure 7. As shown in the measured accelerometry, triaxial accelerometer values may be recorded and the angular information may be derived from the triaxial accelerometer values of the measured accelerometry. As it is shown in Figure 7, episodes of apnea detected recorded in the polysomnography can be identified by the system as well. Dotted boxes represent examples of apnea. Similarly, episodes of hypopnea and snoring can be identified. Gray boxes represent hypopnea and arrows represent snoring.

Figure 8 shows apnea and hypopnea types detected by the system according to an embodiment. When a silence event is detected, the silence event can be classified as either apnea or hypopnea. If there are breathing or snoring sounds in the audio signal, the event may be classified a hypopnea, while if there is only silence or artifacts, the event may be classified as an apnea.

As it can be shown in Figure 7 and Figure 8 and understood from the above explanation, the wording of the silence event may not be limited to an event where there is no sound detected at all. Since silence events can be recognized based on sample entropy values, an event with a sample entropy value smaller than a threshold may be identified as a silence event. The silence event with the sample entropy smaller than the threshold may imply an existence of low-intensity respiratory sounds in the audio signals.

In the episodes of silence events shown in Figure 7 and Figure 8, there are audio signals indicating existence of breathing or snoring sounds. Based on this sound and the respective angular information, events can be classified in different types. For example, in hypopneas and obstructive apneas, there is a reduction in the thoracic movement as indicated by the angular variation derived from triaxial accelerometer data. In central apneas, there is no thoracic movement as indicated by flat regions in the angular information.

Figure 9 shows differences between nasal breathing and oral breathing. According to an embodiment, it is possible to distinguish different breathing routes based on spectral content of audio signals. Top panels of Figure 9 show the signals in the time domain and lower plots show their respective Fast Fourier Transform (FFT) with envelopes in dotted lines. Since unlike nasal breathing, oral breathing has a prominent peak at higher frequencies, i.e. around 1.5 kHz, nasal breathing and oral breathing can be distinguished by the system.

Figure 10 shows cardiac activity measured by the accelerometer of the system according to an embodiment, compared to an electrocardiogram (ECG) recorded by classical polysomnography. The upper plot of Figure 10 shows the ECG from the classical polysomnography, while the lower plot shows movements along z-axis measured by the accelerometer of the system, including peaks corresponding to cardiac activity. Therefore, the accelerometer of the system can identify cardiac activity.

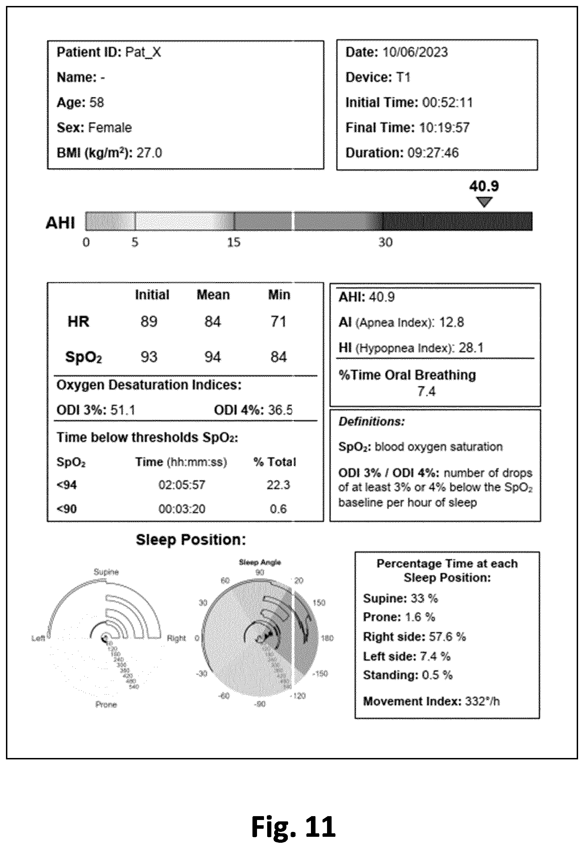

Figure 11 shows an example of a report which can be made by the system according to an embodiment. For example, the report may be shown in or prepared to be downloaded from the smartphone application of the mobile device.

In the report shown in Figure 11, AHI stands for Apnea-Hypopnea Index which is the number of silence events, that is, the number of episodes of absence or significant reduction of the respiratory flow per hour of sleep. The absence of the respiratory flow may imply an apnea and the significant reduction of the respiratory flow may imply a hypopnea.

The method according to the present disclosure can be used for different use cases such as healthy sleep quality monitoring in general population, early detection of sleep disorders, sleep apnea monitoring, longitudinal follow up and personalized positional therapy delivery.

The present disclosure would benefit different types of users including healthy subjects, that is, general population, and patients with sleep apnea. Also, the present disclosure would benefit patents with comorbidities leading to an increased risk of sleep disorders such as patients with spinal cord injury, post-stroke patients, chronic obstructive pulmonary disease (COPD) patients and patients with other respiratory diseases, neurological disorders, or mental disorders, among others.

Although detailed embodiments have been described, these only serve to provide a better understanding of the invention defined by the independent claims and are not to be seen as limiting.

### Reference Signs

- A: audio signals
- P, P': subject person
- 10: mobile device
- 101: microphone
- 102: accelerometer
- 2: fixation system
- 21: elastic band
- 22: bag
- 3: pulse oximeter
- 4: system

## Claims

1. A system for monitoring a sleeping disorder in a subject person, the system comprising:
a mobile device comprising:
a microphone configured to acquire audio signals indicative of breathing of the subject person; and
an accelerometer configured to acquire accelerometer signals including position, angular and movement information of the subject person;
a fixation system for collocating the mobile device above the sternum of the subject person; and
a pulse oximeter configured to measure oxygen saturation levels of the subject person, wherein
the fixation system is composed of an elastic band and a bag to place the mobile device;
wherein the system is configured for monitoring a sleeping disorder in a subject person by:
- acquiring audio signals indicative of breathing of the subject person by means of the microphone;
- acquiring accelerometer signals, by means of the accelerometer of the mobile device, to derive position, angular and movement information of the subject person from the accelerometer signals;
- measuring thoracic movement related to respiration and sleep-disordered breathing by means of the accelerometer;
- identifying a plurality of silence events based on the audio signals;
- classifying the silence events into apnea or hypopnea depending on existence of low-intensity respiratory sounds in the audio signals;
- identifying an apnea type of the classified apnea; and
- associating to a classified silence event the respective angular information by combining the audio signals and the accelerometer signals;
wherein the apnea type is identified based on the measured thoracic movement as indicated by the angular information derived from the accelerometer.

2. The system according to claim 1, wherein the apnea type is identified as obstructive, central, or mixed by means of the angular information derived from the accelerometer; and
wherein a reduction in the thoracic movement as indicated by the angular variation derived from triaxial accelerometer data comprised in the angular information is identified as an obstructive apnea,
wherein an absence of thoracic movement as indicated by flat regions in the angular information is identified as a central apnea, and
wherein an apnea which begins as a central apnea and ends as an obstructive apnea or vice versa is identified as a mixed apnea

3. The system according to any one of the preceding claims, wherein the system is configured for monitoring a sleeping disorder in a subject person by further:
- quantifying sleep movements and sleep position variability based on the accelerometer signals; and
- detecting breathing route during sleep from the audio signals.

4. The system according to any one of the preceding claims, wherein the pulse oximeter is a wireless pulse oximeter and wherein the system is configured for monitoring a sleeping disorder in a subject person by further:
- acquiring oxygen saturation levels of the subject person by means of the wireless pulse oximeter via a wireless link to the mobile device; and
- selecting regions of interest, wherein the regions in interest are regions of the audio signals that are followed by oxygen desaturations measured by the wireless pulse oximeter.

5. The system according to any one of the preceding claims, wherein the system is configured for monitoring a sleeping disorder in a subject person by further:
- removing artefacts by considering the accelerometer signals while identifying the silence events.

6. The system according to any one of the preceding claims, wherein the system is configured for monitoring a sleeping disorder in a subject person by further:
- providing the classified silence events with associated the respective angular information on the mobile device.

7. The system according to any one of the preceding claims, wherein the system is configured for monitoring a sleeping disorder in a subject person by further:
- outputting vibration and/or sound from the mobile device based on the angular information and the oxygen saturation level and/or the audio signals for positional treatment.

8. The system according to any one of the preceding claims, wherein
the apnea type is obstructive, central, or mixed;
the breathing route is oral or nasal; and
the angular information is one angle, a plurality of angles, or triaxial accelerometer values.

9. The system according to any one of the preceding claims, wherein the system is configured for monitoring a sleeping disorder in a subject person by further:
- detecting cardiac activity from the accelerometer; and
- associating a relationship of changes of heart rate of the subject person and silence events.

## Patentansprüche

1. System zum Überwachen einer Schlafstörung in einem menschlichen Individuum, wobei das System Folgendes umfasst:
ein Mobilgerät umfassend:
ein Mikrophon, welches dazu ausgebildet ist, Tonsignale hinweisend auf die Atmung des menschlichen Individuums zu erfassen; und
einen Beschleunigungsmesser, welcher dazu ausgebildet ist, Beschleunigungsmessersignale einschließlich Stellungs-, Winkel- und Bewegungsinformation des menschlichen Individuums zu erfassen;
ein Fixierungssystem zum Platzieren des Mobilgeräts über dem Sternum des menschlichen Individuums; und
ein Pulsoximeter, welches dazu ausgebildet ist, Sauerstoffsättigungsgrade des menschlichen Individuums zu messen, wobei
das Fixierungssystem aus einem elastischen Band und einem Beutel zum Platzieren des Mobilgeräts besteht;
wobei das System zum Überwachen einer Schlafstörung in einem menschlichen Individuum ausgebildet ist, indem es:
- Tonsignale hinweisend auf die Atmung des menschlichen Individuums mittels des Mikrophons erfasst;
- Beschleunigungsmessersignale, mittels des Beschleunigungsmessers des Mobilgeräts erfasst, um Stellungs-, Winkel- und Bewegungsinformation des menschlichen Individuums von den Beschleunigungsmessersignalen abzuleiten;
- die Bewegung des Brustkorbs, welche mit einer Atmung und einer schlafgestörten Atmung verbunden ist, mittels des Beschleunigungsmessers misst;
- eine Vielzahl von Stilleereignissen basierend auf den Tonsignalen identifiziert;
- die Stilleereignisse als Apnoe oder Hypopnoe in Abhängigkeit des Vorhandenseins von Atmungsgeräuschen niedriger Intensität in den Tonsignalen einstuft;
- einen Apnoetyp der eingestuften Apnoe identifiziert; und
- die jeweilige Winkelinformation mit einem eingestuften Stilleereignis assoziiert, indem es die Tonsignale und die Beschleunigungsmessersignale kombiniert;
wobei der Apnoetyp basierend auf der gemessenen Bewegung des Brustkorbs, wie durch die vom Beschleunigungsmesser abgeleitete Winkelinformation angezeigt, identifiziert wird.

2. System nach Anspruch 1, wobei der Apnoetyp als obstruktiv, zentral oder gemischt mittels der vom Beschleunigungsmesser abgeleiteten Winkelinformation identifiziert wird; und
wobei eine Verringerung in der Bewegung des Brustkorbs, wie durch die von den triaxialen Beschleunigungsmesserdaten, welche in der Winkelinformation umfasst sind, abgeleitete Winkelvariation angezeigt, als obstruktive Apnoe identifiziert wird,
wobei eine Abwesenheit von Bewegung des Brustkorbs, wie durch flache Bereiche in der Winkelinformation angezeigt, als zentrale Apnoe identifiziert wird, und
wobei eine Apnoe, welche als zentrale Apnoe anfängt und als obstruktive Apnoe endet, oder umgekehrt, als gemischte Apnoe identifiziert wird.

3. System nach einem der vorhergehenden Ansprüche, wobei das System zum Überwachen einer Schlafstörung in einem menschlichen Individuum ausgebildet ist, indem es zusätzlich:
- Schlafbewegungen und die Variabilität der Schlafstellung basierend auf den Beschleunigungsmessersignalen quantifiziert; und
- die Atemroute während des Schlafs aus den Tonsignalen detektiert.

4. System nach einem der vorhergehenden Ansprüche, wobei das Pulsoximeter ein drahtloses Pulsoximeter ist und wobei das System zum Überwachen einer Schlafstörung in einem menschlichen Individuum ausgebildet ist, indem es zusätzlich:
- Sauerstoffsättigungsgrade des menschlichen Individuums mittels des drahtlosen Pulsoximeters über eine drahtlose Verbindung zum Mobilgerät erfasst; und
- Bereiche von Interesse auswählt, wobei die Bereiche von Interesse Bereiche der Tonsignale sind, welche von durch das drahtlose Pulsoximeter gemessenen Sauerstoffuntersättigungen gefolgt werden.

5. System nach einem der vorhergehenden Ansprüche, wobei das System zum Überwachen einer Schlafstörung in einem menschlichen Individuum ausgebildet ist, indem es zusätzlich:
- Artefakte beseitigt, indem die Beschleunigungsmessersignale während des Identifizierens der Stilleereignisse berücksichtigt werden.

6. System nach einem der vorhergehenden Ansprüche, wobei das System zum Überwachen einer Schlafstörung in einem menschlichen Individuum ausgebildet ist, indem es zusätzlich:
- die eingestuften Stilleereignisse mit jeweiliger assoziierter Winkelinformation auf dem Mobilgerät bereitstellt.

7. System nach einem der vorhergehenden Ansprüche, wobei das System zum Überwachen einer Schlafstörung in einem menschlichen Individuum ausgebildet ist, indem es zusätzlich:
- Vibration und/oder Ton vom Mobilgerät basierend auf der Winkelinformation und dem Sauerstoffsättigungsgrad und/oder den Tonsignalen für die Lagerungstherapie ausgibt.

8. System nach einem der vorhergehenden Ansprüche, wobei
der Apnoetyp obstruktive, zentral oder gemischt ist;
die Atemroute oral oder nasal ist; und
die Winkelinformation ein Winkel, eine Vielzahl von Winkeln oder triaxiale Beschleunigungsmesserwerte ist.

9. System nach einem der vorhergehenden Ansprüche, wobei das System zum Überwachen einer Schlafstörung in einem menschlichen Individuum ausgebildet ist, indem es zusätzlich:
- die Herzaktivität aus dem Beschleunigungsmesser detektiert; und
- ein Verhältnis der Änderungen der Herzfrequenz des menschlichen Individuums und der Stilleereignisse assoziiert.

## Revendications

1. Système pour surveillance d'un trouble du sommeil chez un sujet humain, le système comprenant :
un dispositif mobile comprenant :
un microphone configuré pour acquérir des signaux d'audio indicatifs de la respiration du sujet humain ; et
un accéléromètre configuré pour acquérir des signaux d'accéléromètre comportant des informations de position, angulaires et de mouvement du sujet humain ;
un système de fixation pour placer le dispositif mobile sur le sternum du sujet humain ; et
un oxymètre de pouls configuré pour mesurer les niveaux de saturation en oxygène du sujet humain, dans lequel
le système de fixation est composé d'une bande élastique et d'un sac pour placer le dispositif mobile ;
dans lequel le système est configuré pour surveiller un trouble du sommeil chez un sujet humain par le biais :
- d'acquérir des signaux d'audio indicatifs de la respiration du sujet humain au moyen du microphone ;
- d'acquérir des signaux d'accéléromètre, au moyen de l'accéléromètre du dispositif mobile, pour dériver des informations de position, angulaires et de mouvement du sujet humain à partir des signaux d'accéléromètre ;
- de mesurer le mouvement thoracique lié à la respiration et à une respiration présentant un trouble du sommeil au moyen de l'accéléromètre ;
- d'identifier une pluralité d'événements de silence en fonction des signaux d'audio ;
- de classifier les événements de silence en apnée ou hypopnée en fonction de l'existence de sons respiratoires de faible intensité dans les signaux d'audio ;
- d'identifier un type d'apnée de l'apnée classifiée ; et
- d'associer les informations angulaires respectives à un événement de silence classifié par combinaison des signaux d'audio et des signaux d'accéléromètre ;
dans lequel le type d'apnée est identifié en fonction du mouvement thoracique mesuré tel qu'indiqué par les informations angulaires dérivées à partir de l'accéléromètre.

2. Système selon la revendication 1, dans lequel le type d'apnée est identifié comme obstructive, centrale, ou mixte au moyen des informations angulaires dérivées à partir de l'accéléromètre ; et
dans lequel une réduction du mouvement thoracique telle qu'indiquée par la variation angulaire dérivée à partir de données d'accéléromètre triaxiales compris dans les informations angulaires est identifiée comme une apnée obstructive,
dans lequel une absence de mouvement thoracique telle qu'indiquée par des régions plates dans les informations angulaires est identifiée comme une apnée centrale, et
dans lequel une apnée qui commence comme une apnée centrale et se termine comme une apnée obstructive ou vice versa est identifiée comme une apnée mixte.

3. Système selon l'une quelconque des revendications précédentes, dans lequel le système est configuré pour surveiller un trouble du sommeil chez un sujet humain par le biais, en outre :
- de quantifier des mouvements de sommeil et la variabilité de position de sommeil en fonction des signaux d'accéléromètre ; et
- de détecter la voie de respiration pendant le sommeil à partir des signaux d'audio.

4. Système selon l'une quelconque des revendications précédentes, dans lequel l'oxymètre de pouls est un oxymètre de pouls sans fil et dans lequel le système est configuré pour surveiller un trouble du sommeil chez un sujet humain par le biais, en outre :
- d'acquérir des niveaux de saturation en oxygène du sujet humain au moyen de l'oxymètre de pouls sans fil à travers une liaison sans fil avec le dispositif mobile ; et
- de choisir des régions d'intérêt, dans lequel les régions d'intérêt sont des régions des signaux d'audio qui sont suivies par des désaturations en oxygène mesurées par l'oxymètre de pouls sans fil.

5. Système selon l'une quelconque des revendications précédentes, dans lequel le système est configuré pour surveiller un trouble du sommeil chez un sujet humain par le biais, en outre :
- d'éliminer des artefacts en considérant les signaux d'accéléromètre lors de l'identification des événements de silence.

6. Système selon l'une quelconque des revendications précédentes, dans lequel le système est configuré pour surveiller un trouble du sommeil chez un sujet humain par le biais, en outre :
- de fournir les événements de silence classifiés avec les informations angulaires respectives associées sur le dispositif mobile.

7. Système selon l'une quelconque des revendications précédentes, dans lequel le système est configuré pour surveiller un trouble du sommeil chez un sujet humain par le biais, en outre :
- d'émettre des vibrations et/ou du son à partir du dispositif mobile en fonction des informations angulaires et du niveau de saturation en oxygène et/ou des signaux d'audio pour le traitement de la position.

8. Système selon l'une quelconque des revendications précédentes, dans lequel
le type d'apnée est obstructive, centrale, ou mixte ;
la voie de respiration est orale ou nasale ; et
les informations angulaires sont un angle, une pluralité d'angles, ou des valeurs d'accéléromètre triaxiales.

9. Système selon l'une quelconque des revendications précédentes, dans lequel le système est configuré pour surveiller un trouble du sommeil chez un sujet humain par le biais, en outre :
- de détecter l'activité cardiaque à partir de l'accéléromètre ; et
- d'associer une relation de changements de la fréquence cardiaque du sujet humain et des événements de silence.
